Europäisches Patentamt

(19) European Patent Office (11) Publication number: **0 041 403**

Office européen des brevets **B1**

(12) **EUROPEAN PATENT SPECIFICATION**

(45) Date of publication of patent specification: **05.12.84** (51) Int. Cl.³: **G 01 S 15/89,** G 01 S 7/52, A 61 B 10/00

(21) Application number: **81302455.1**

(22) Date of filing: **03.06.81**

(54) **Method and apparatus for ultrasonic wave measurement of characteristics of the internal structure of an object.**

(30) Priority: **03.06.80 JP 74680/80**

(43) Date of publication of application:
**09.12.81 Bulletin 81/49**

(45) Publication of the grant of the patent:
**05.12.84 Bulletin 84/49**

(84) Designated Contracting States:
**DE FR GB**

(56) References cited:
**US-A-3 156 110**
**US-A-3 603 919**
**US-A-4 057 049**
**US-A-4 176 658**
**US-A-4 228 804**

**IEEE TRANSACTIONS ON BIOMEDICAL ENGINEERING, vol. BME-25, no. 4, July 1978, IEEE, NEW YORK (US) A.C. KAK et al.: "Signal Processing of Broadband Pulsed Ultrasound: Measurement of Attenuation of Soft Biological Tissues", pages 321-344**

The file contains technical information submitted after the application was filed and not included in this specification

(73) Proprietor: **FUJITSU LIMITED**
**1015, Kamikodanaka Nakahara-ku**
**Kawasaki-shi Kanagawa 211 (JP)**

(72) Inventor: **Miwa, Hirohide**
**6-7-10, Miyazaki, Takatsu-ku**
**Kawasaki-shi Kanagawa 213 (JP)**
Inventor: **Shimura, Takaki**
**29-44, Tsurukawa 4-chome**
**Machida-shi Tokyo 194-01 (JP)**
Inventor: **Murakami, Keiischi**
**1259-8, Kamikodanaka, Nakahara-ku**
**Kawasaki-shi Kanagawa 211 (JP)**

(74) Representative: **Sunderland, James Harry et al**
**HASELTINE LAKE & CO Hazlitt House 28**
**Southampton Buildings Chancery Lane**
**London WC2A 1AT (GB)**

(56) References cited:
**ACOUSTICAL IMAGING, vol. 9: Visualization and Characterization 1979, Plenum Press, Editor: K.Y. WANG NEW YORK (US) J.P. JONES et al.: "A computerized data analysis system for ultrasonic tissue characterization", pages 503-512**

**IEEE TRANSACTIONS ON NUCLEAR SCIENCE,
vol. NS-27, no. 3, June 1980, IEEE J.P. JONES:
"Quantitative Characterization of Tissue using
Ultrasound", pages 1168-1175**

## Description

The present invention relates to method and apparatus for ultrasonic wave measurement of characteristics of the internal structure of an object, for example the human body.

In the fields of medical diagnostic technology and metal flaw detector technology etc., ultrasonic wave image technology is widely employed. In ultrasonic wave image technology, the condition of a domain within an object, for example, or the location of a boundary with respect to a beam transmitting location, is displayed by transmitting an ultrasonic wave beam to the object and receiving and analyzing a wave reflected from the boundary of the domain within the object. A wave transmitted in an object is attenuated in accordance with the characteristics of the internal structure of the object and is returned as a reflected wave after suffering reflection, in accordance with a reflection coefficient, at a boundary in the object.

Therefore, the attenuation characteristics of the internal structure of an object can be measured or determined by analysing such a reflected wave in relation to the transmitted wave.

Such technology is mentioned in Japanese laid-open patent specification No. 49-38490 or in Japanese published patent specification No. 5224798.

These documents describe the emission of an ultrasonic wave of a plurality of frequencies, the sonic pressure of each frequency in a received reflected wave is obtained, and then the characteristics of attenuation of an internal structure are obtained in dependence upon the ratio of such sonic pressures.

US—A—4 057 049 discloses a similar system in which characteristics are determined in dependence upon the pressure-amplitude of received reflected ultrasonic waves.

IEEE Transactions on Biomedical Engineering, Vol. DME-25, No. 4, July 1978, at pages 321 to 344, discloses a system in which characteristics are determined from ultrasonic waves received after direct transmission through an object. The use of the ratio of energies of received ultrasonic waves of different frequencies for determining attenuation of the object through which the waves have been directly transmitted is mentioned.

Sonic pressure or pressure amplitude systems, and direct transmission systems, suffer major problems. For example, in sonic pressure or pressure amplitude systems so-called phase cancellation is a major problem. The result is that an accurate attenuation characteristic cannot be obtained.

According to one aspect of the present invention there is provided an ultrasonic wave method of measuring characteristics of attenuation of a domain or domains in an object, by transmitting an ultrasonic wave into the object and by receiving a reflected ultrasonic wave from the object, comprising:—

transmitting ultrasonic waves of a plurality of different frequencies into the object,

receiving ultrasonic waves reflected from the object, and

measuring characteristics of attenuation of the domain or domains from the ratio or ratios of intensities of received reflected ultrasonic waves corresponding to the respective different frequencies of the plurality.

According to another aspect of the present invention there is provided ultrasonic wave apparatus for measuring characteristics of attenuation of a domain or domains in an object, by transmitting an ultrasonic wave into the object and by receiving a reflected ultrasonic wave from the object, comprising:—

ultrasonic wave transmit/receive means for transmitting ultrasonic waves of a plurality of different frequencies into the object and for receiving ultrasonic waves reflected from the object, and

measurement means for measuring characteristics of attenuation of the domain or domains from the ratio or ratios of intensities of received reflected ultrasonic waves corresponding to the respective different frequencies of the plurality.

An embodiment of the present invention can provide an ultrasonic wave measurement method for accurately measuring attenuation characteristics.

An embodiment of the present invention can provide an ultrasonic wave measurement method for measuring attenuation characteristics which does not suffer the major problems experienced with the prior art.

An embodiment of the present invention can provide an ultrasonic wave measurement method for easily measuring attenuation characteristics.

An embodiment of the present invention can provide an ultrasonic wave measurement method suitable for measuring attenuation characteristics of the internal structure of the human body.

An embodiment of the present invention can provide an ultrasonic wave measurement method for measuring attenuation characteristics on the basis of reflected signal intensity.

According to an embodiment of the present invention, an ultrasonic wave containing a plurality of frequencies is transmitted and a reflected wave obtained from an object is received, in accordance with the ultrasonic wave transmitted. The intensity of a reflected wave, namely the energy of a reflected wave, is used for measurement, and the ratio of intensities of reflected waves corresponding to respective transmission frequencies determines the attenuation characteristics of the object.

Since reflected wave energy is not influenced by factors such as phase cancellation which give

0 041 403

rise to the major problems experienced with the prior art, it is essential to receive a reflected wave containing accurate attenuation characteristic and to output intensity of the reflected wave instead of sonic pressure.

For this purpose, an acousto-electric element such as CdS is used as a receiving transducer, or a piezoelectric element such as PZT etc so structured as to have divided receiving surfaces and used to can generate an intensity output by the provision of square-calculation circuitry.

Typical attenuation characteristics thus obtained are the attenuation coefficient and the attenuation slope and these may be displayed as an attenuation characteristic distribution diagram of an object on a display screen.

Reference is made, by way of example, to the accompanying drawings, in which:—

Figures 1 and 2 are respective schematic cross-sectional diagrams for assistance in explaining the principle of the present invention,

Figure 3 is a waveform diagram illustrating the inter-relationship between transmitting and receiving signals in an embodiment of the present invention,

Figure 4 is a schematic block diagram of apparatus embodying the present invention,

Figure 5 is a more detailed block diagram of a receiving circuit of the apparatus of Figure 4,

Figure 6 is a more detailed block diagram of an arithmetic operation circuit of the receiving circuit of Figure 5,

Figure 7 is a more detailed block diagram of a display control circuit of the apparatus of Figure 4, and

Figure 8 is a distribution diagram of attenuation characteristics indicated by an embodiment of the present invention.

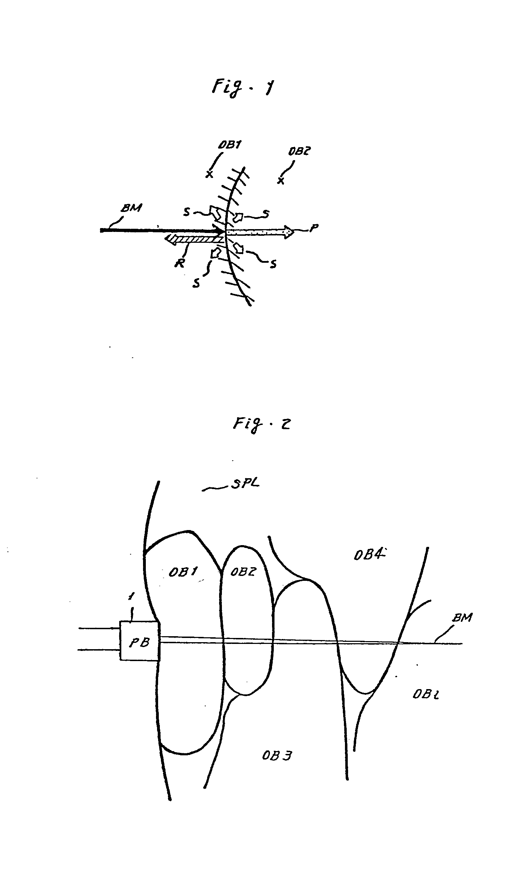

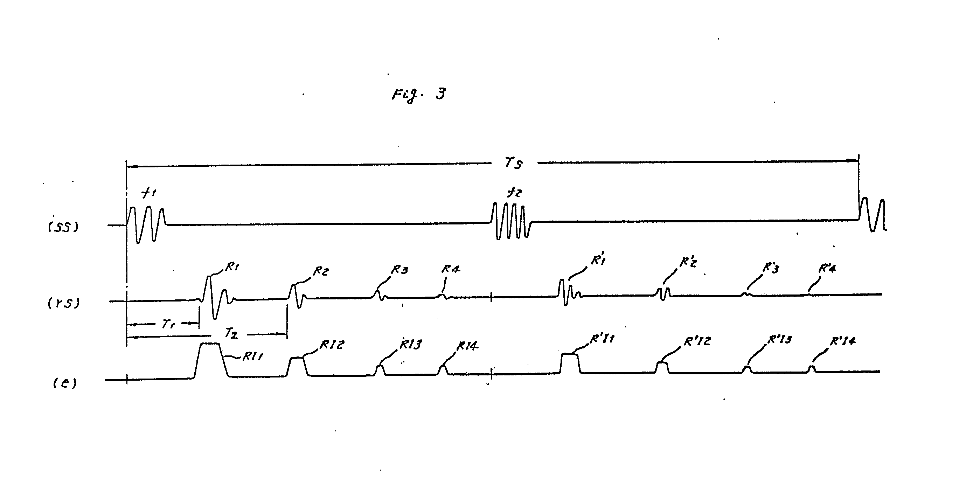

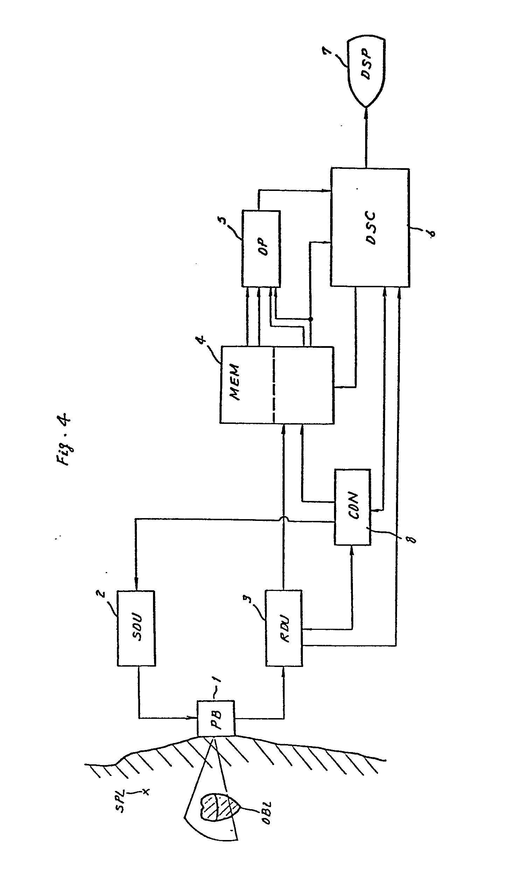

Figure 1 and Figure 2 are respective cross-sections of an object, for assistance in explaining the principle of the present invention. Figure 3 is a waveform diagram in the form of a time chart.

In Figures 1 and 2, OB1 to OBi are domains, SPL is a specimen used as an object, PB is a transmit-receive element for ultrasonic wave transmission and reception (hereinafter referred to as transducer), BM is a transmitted ultrasonic wave.

In Figure 1, the ultrasonic wave BM travelling through the domain OB1 is generally subjected to energy attenuation in the domain OB1 and then reflected and scattered at the boundary between the domains OB1 and OB2, but partly penetrating the boundary. If the intensity transmissivity at the OB1— OB2 boundary is taken to be P, the intensity reflection coefficient at the boundary to be R and the intensity scattering ratio, combining all scattering directions, to be S, the following relationship exists between the transmissivity, the reflection coefficient and the scattering ratio.

$$P+R+S=1$$

In regard to the above equation, it is known that the transmissivity, the reflection coefficient and the scattering ratio vary generally in accordance with the incident angle for the domain OB2 and with the geometry of the boundary surface, but the above equation is satisfied for different frequencies of transmitted ultrasonic waves.

Moreover, if the ultrasonic wave BM were travelling in a direction opposite to that of Figure 1, the above equation should still be satisfied.

In addition, the attenuation of ultrasonic wave energy in a domain is expressed as an exponential function of path length. On the other hand, an attenuation coefficient is generally a function of ultrasonic wave frequency—an almost linear function of frequency within a specified frequency range. Moreover, ultrasonic wave propagation velocity does not depend on the ultrasonic wave frequency.

The principle of the present invention will be explained using Figure 2 and Figure 3, on the basis of these pre-conditions for ultrasonic waves.

First, symbols are defined as indicated below.

Intensity of transmitted ultrasonic wave: $I$

Intensity of reflected wave which is reflected from the boundary between the domains OBi and OBi+1 and received at the transmitting location (hereinafter referred to as reflected intensity): $RIi\ (i=1,2\dots)$

Intensity attenuation coefficient of domain OBi: $\mu i\ (i=1,2\dots)$

Path length of domain OBi: $li\ (i=1,2\dots)$

Intensity transmissivity at the boundary between the domains OBi and OBi+1: $Pi\ (i=1,2\dots)$

Intensity reflection coefficient: $Ri\ (i=1,2\dots)$

Intensity scattering ratio: $Si\ (i=1,2\dots)$

Ultrasonic wave propagation velocity in the domain OBi: $vi\ (i=1,2\dots)$

Here, intensity means a generally used instantaneous or time-integrated energy.

For explaining the principles of the present invention two frequencies $f_1$, $f_2$ of ultrasonic wave are used and the attenuation coefficient $\mu i$ and intensity $I$ corresponding to the frequency $f_2$ will be

4

distinguished from those corresponding to the frequency $f_1$ by a prime mark at the upper right side of the symbols.

The reflection coefficient Ri, the transmissivity Pi and the scattering ratio Si are the same respectively for frequencies $f_1$, $f_2$ because in substance they do not depend on frequency at a specular boundary.

Ultrasonic wave beams of frequencies $f_1$ and $f_2$ are transmitted as indicated at waveform (SS) in Figure 3 from the transducer PB.

The $f_1$ frequency ultrasonic wave beam BM is reflected at the boundary between the domains OBi and OBi+1 (i=1 to 4) and the reflected waves $R_1$, $R_2$, $R_3$, $R_4$ indicated at waveform (rs) of Figure 3 are received. These reflected waves correspond to reflections at the mutual boundaries of OB1, OB2, OB3, OB4. The intensities RI1, RI2 of signals $R_1$, $R_2$ are expressed as follows, in accordance with the explanation given previously.

$$RI1 = I \cdot \exp(-\mu_1 \cdot I_1) \cdot R_1 \cdot \exp(-\mu_1 \cdot I_1) = I \cdot R_1 \cdot \exp(-2\mu_1 \cdot I_1)$$

$$RI2 = I \cdot \exp(-\mu_1 \cdot I_1) \cdot P_1 \cdot \exp(-\mu_2 \cdot I_2) \cdot R_2 \cdot \exp(-\mu_2 \cdot I_2) \cdot \overline{P}_1 \cdot \exp(-\mu_1 \cdot I_1)$$

$$= I \cdot R_2 \cdot \exp\{-2(\mu_1 \cdot I_1 + \mu_2 \cdot I_2)\} \cdot P_1 \, \overline{P}_1$$

The general equation is expressed as follows.

$$RIi = I \cdot Ri \cdot \prod_{m=1}^{i} \{\exp(-2\mu_m \cdot I_m) \cdot P_{m-1} \cdot \overline{P}_{m-1}\} \tag{1}$$

Where, $P_0 = 1$.

Moreover, the intensity R'Ii of the signal R'i in a case in which an ultrasonic wave beam of frequency $f_2$ is transmitted from the transducer PB is expressed by the following general equation.

$$R'Ii = I' \cdot Ri \cdot \prod_{m=1}^{i} \{\exp(-2\mu'_m \cdot I_m) \cdot P_{m-1} \cdot \overline{P}_{m-1}\} \tag{2}$$

(P may be understood to be transmittivity going one way through a domain boundary, and $\overline{P}$ may be understood to be transmittivity going the other way through the boundary).

Therefore, the ratio of intensity I of ultrasonic wave transmitted and the received reflected signal intensity RI (at frequency $f_1$) is expressed as follows using natural logarithms $(1_n)$.

For the frequency $f_1$,

$$1_n(RI_1/I) = -2\mu_1 \cdot I_1 + 1_n(R_1)$$
$$1_n(RI_2/I) = -2\mu_1 \cdot I_1 - 2\mu_2 \cdot I_2 + 1_n(R_2) + 1_n(\overline{P}_1 \cdot P_1)$$

$$1_n(RI_i/I) = \sum_{m=1}^{i} \{-2\mu_m \cdot I_m + 1_n(P_{m-1} \cdot \overline{P}_{m-1})\} + 1_n(R_i) \tag{3}$$

In the same way, the generation equation for the frequency $f_2$ is as follows.

$$1_n(R'I_i/I') = \sum_{m=1}^{i} \{-2\mu'_m \cdot I_m + 1_n(P_{m-1} \cdot \overline{P}_{m-1})\} + 1_n(R_i) \tag{4}$$

Here, when a difference between equations (3) and (4) is obtained, it is expressed by the following equation.

$$1_n(RI_i/I) - 1_n(R'I_i/I') = \sum_{m=1}^{i} (-2\mu_m \cdot I_m) - \sum_{m=1}^{i} (-2\mu'_m \cdot I_m)$$

$$= \sum_{m=1}^{i} -2I_m \cdot (\mu_m - \mu'_m) \tag{5}$$

The left side of equation (5) is obtained by measuring the intensities I, I′ of ultrasonic waves of frequencies $f_1$, $f_2$ and the reflected intensities RI, R′I. Here, $1_m$ on the right side of equation (5) is obtained as follows.

When propagation velocity vi in domain OBi is used, the period Ti from the time of transmission of the ultrasonic wave signal I to the time of receiving the reflected wave Ri is expressed by the following equations respectively for domains OB1, OB2, etc.

$$T_1 = \frac{2l_1}{V_1}$$

$$T_2 = \frac{2l_1}{V_1} + \frac{2l_2}{V_2}$$

$$\vdots$$

$$T_i = 2 \sum_{m=1}^{i} \frac{2l_m}{V_m}$$

Therefore,

$$1_n(RI_1/I) - 1_n(R'I_1/I') = -T_1 \cdot V_1 \cdot (\mu_1 - \mu'_1)$$
$$1_n(RI_2/I) - 1_n(R'I_2/I') = -T_1 \cdot V_1 \cdot (\mu_1 - \mu'_1) - (T_2 - T_1) \cdot V_2 \cdot (\mu_2 - \mu'_2)$$
$$= 1_n(RI_1/I) - 1_n(R'I_1/I') - (T_2 - T_1) \cdot V_2 \cdot (\mu_2 - \mu'_2) \tag{6}$$

The general equation is expressed as follows.

$$1_n(RI_i/I) - 1_n(R'I_i/I_i) = -V_i(T_i - T_{i-1}) \cdot (\mu_i - \mu_i') + 1_n(RI_{i-1}/I) - 1_n(R'I_{i-1}/I') \tag{7}$$

In addition, the following equation can be obtained from equation (7).

$$1_n\left(\frac{RIi}{R'Ii} \cdot \frac{I'}{I}\right) = -V_i \cdot (T_i - T_{i-1}) \cdot (\mu_i - \mu'_i) + 1_n\left(\frac{RI_{i-1}}{R'I_{i-1}} \cdot \frac{I'}{I}\right)$$

$$\therefore V_i \cdot (\mu_i - \mu'_i) = \left\{1_n\left(\frac{RI_{i-1}}{R'I_{i-1}} \cdot \frac{I'}{I}\right) - 1_n\left(\frac{RI_i}{R'I_i} \cdot \frac{I'}{I}\right)\right\} / (T_i - T_{i-1}) \tag{8}$$

However, since the transmission intensities I, I′ are known and the reflected receiving signal intensities RIi, R′Ii, RIi—1, R′Ii—1 and times Ti, Ti—1 can be determined by actual measurement, the value Vi $\cdot (\mu_i - \mu'_i)$ can be obtained by arithmetic operation.

In equation (8), propagation velocity in the internal structure can be approximated almost to a constant value $v_0$ and when the intensities I, I′ of beams of respective frequencies are constant, $I_0$, the value $(\mu_i - \mu'_i)$ can be simplified as follows.

For example for i=1

$$\mu_1 - \mu'_1 = \frac{1n(RI_1/R'I_1)}{T_1 \cdot V_0}$$

The general expression is as follows.

$$\mu_i - \mu'_i = -\frac{1}{V_0} \cdot \frac{1}{T_i - T_{i-1}} \left(1_n\frac{RI_i}{R'I_i} - 1_n\frac{RI_{i-1}}{R'I_{i-1}}\right) \tag{9}$$

Namely, when the reflected receiving signal intensities RI, R′I, for transmitted waves of respective frequencies, and the times T are measured, the value of $(\mu_i - \mu'_i)$ can be obtained since the propagation velocity $v_0$ is known.

Moreover, the attenuation slope $\alpha$ can be obtained as indicated below. It is known that when the attenuation slope $\alpha$ is expressed in terms of the transmitting frequency f(MHz) and the attenuation coefficient $\mu$, the attenuation slope $\alpha$ (db/cm.MHz) used in general becomes as follows.

6

$$\alpha = 4.343 \cdot \frac{\mu}{f}$$

Therefore, when the value $(\mu_i - \mu'_i)$ obtained by calculation is used, the attenuation slope $\alpha$ can be expressed as follows.

$$\alpha = 4.343 \times \frac{\mu_i - \mu'_i}{f_1 - f_2} \qquad (10)$$

Therefore, the following equation can be obtained by substituting equation (9) into equation (10).

$$\alpha = \frac{1}{V_0} \cdot 4.343 \cdot (f_1 - f_2)^{-1} \cdot (T_i - T_{i-1})^{-1} \cdot (1_n \frac{RI_i}{R'I_i} - 1_n \frac{RI_{i-1}}{R'I_{i-1}}) \qquad (11)$$

It is also known that the intensity attenuation slopes $\alpha$ of internal structures of the human body are as follows.

| | |
|---|---|
| Adipose tissue | 0.63 (dB/cm. MHz) |
| Blood | 0.18 (dB/cm. MHz) |
| Liver | 0.94 (dB/cm. MHz) |
| Kidney | 1.0 (dB/cm. MHz) |

It has become possible to define the nature of each domain in a sample SPL from the measured attenuation slope $\alpha$ and the above table. For example, it is possible to detect and identify abnormalities such as cancer organisation etc. In equation (11), $(T_i - T_{i-1})$ is the receiving interval of reflected signals, and it can be generated from an output by providing a means for detecting the receiving interval.

It is more effective when a distribution condition of a sample is identified by displaying attenuation slope $\alpha$ or attenuation coefficient $(\mu_i - \mu'_i)$ or the value $v_i \cdot (\mu_i - \mu'_i)$ as distributed images on a screen.

As explained above, in an embodiment of the present invention the intensity of a reflected wave is employed, namely the instantaneous energy value or the pulse-width time-integrated energy value is employed and therefore an embodiment of the present invention can provide accurate measurement without suffering the major problems experienced in the prior art. The energy value of a reflected wave can be output by various methods. In one method, an acousto-electric element such as CdS, which converts sonic energy into electricity, is used as a receiving transducer. In this case, the energy of a reflected wave can be directly output.

In another method, a piezo electric element such as PZT which converts a sonic pressure into a voltage is used as the receiving transducer. Thereby, the voltage must be converted into an energy representation by a square calculation.

In addition, the influence of phase cancellation can be eliminated perfectly by dividing the receiving surface of the piezo-electric element into many minute segments and by providing square-calculation circuits in correspondence to each segment.

Apparatus embodying the present invention will now be explained with reference to Figures 4 to 8.

In Figure 4, 1 is a transducer, which transmits an ultrasonic wave beam and receives reflected waves, and then scans a domain by changing transmitting direction after each completion of a transmit/receive operation involving a pair of ultrasonic wave frequencies. 2 is a transmit unit, which oscillates the transducer 1 to provide different frequencies. 3 is a receive unit, which discriminates reflected signals from the received output of transducer 1 and outputs data indicating intensity of a received signal and data indicating the time from the received signal to the next reflected signal. 4 is a memory unit which sequentially stores the output data of receive unit 3. 5 is an operation unit which performs calculations in accordance with equations (9) and (11) explained above and outputs data identifying respective domains. 6 is a display control unit which synchronizes with receive timing of receive unit 3 and generates display data concerning a tomographic section on the basis of the data of the abovementioned operation unit 5. 7 is a display unit which displays images on a CRT display device. 8 is a control unit which controls switching of transmission and reception, writing into memory unit 4 and read out timing.

Operations are as explained hereunder.

The control unit 8 generates an output signal which goes to the transmit unit 2 in order to drive the transducer 1 with a signal of frequency $f_1$.

Thereby, the send unit 2 outputs a signal by oscillating at the frequency $f_1$, causing the transducer to send an ultrasonic wave beam of frequency $f_1$ to the specimen SPL.

As the control unit 8 sends the output signal to the transmit unit 2 it simultaneously generates a signal indicating send timing to the receive unit 3.

After receiving the send timing signal, the receive unit 3 starts time-counting for assessing the time until a received reflected signal is detected.

Every time that a signal over a certain threshold is received by the receive unit 3 via the transducer 1 the receive unit 3 considers the signal to be a reflected signal and outputs reflected signal data indicating the intensity of such signal, and time data as counted by the time counting operation, to the memory unit 4.

The reflected signal data and the time data are sequentially stored in the memory unit 4 on a time series basis.

After a specified period from send timing, for example, a period longer than the time required by an ultrasonic wave beam to travel to and back from a section of human body, the control unit 8 generates an output which causes oscillation of the transmit unit 2 at the abovementioned frequency $f_2$. Thereby, the transmit unit 2 causes the transducer 1 to send an ultrasonic wave beam of frequency $f_2$ as explained previously.

Meanwhile, the receive unit 3 stores received data in the memory unit 4 as explained above.

Here, the transducer 1 is not always required to be used in common for transmission and reception—alternatively there may be provided individual means for transmission and reception.

The receive unit 3 may have a structure providing a filter which can discriminate between reflected signals corresponding to a plurality of different frequencies. In this case, it is permitted to transmit simultaneously signals of a plurality of different frequencies from a single transducer.

In this case, a plurality of transducers may be provided corresponding to the different frequencies.

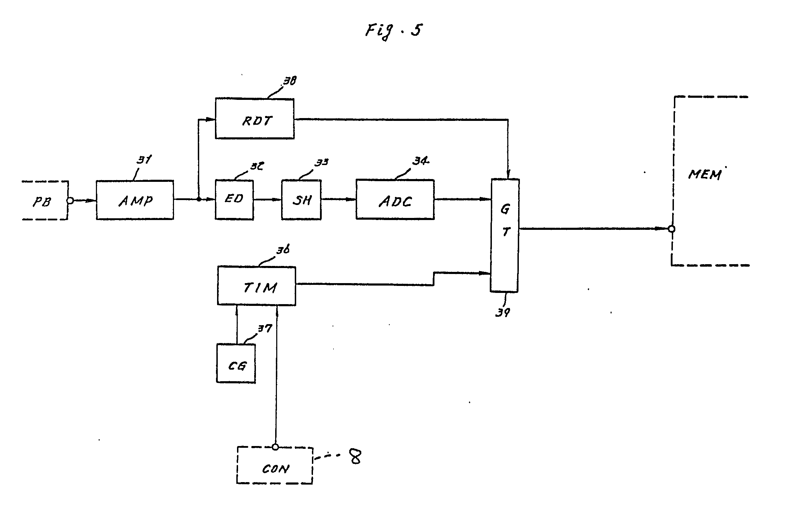

Figure 5 is a more detailed block diagram of the receive unit 3. Operation will be explained below in connection with Figure 5. The received (reflected) signal indicated in waveform (rs) in Figure 3 to be supplied from the transducer 1 is amplified by an amplifier 31. In a case in which attenuation is large, gain is gradually changed from send timing (e.g. gain is progressively increased) so that the greater attenuation of received signals returned from deeper areas is compensated for, and it is recommended to store data concerning adequate compensation amounts in the memory unit.

The amplified received signal is square-rectified in an envelope detector 32 and thereby an envelope signal as seen in waveform (e) of Figure 3 can be generated. The envelope signal (e) is sampled and held at a sample and hold circuit 33 (SH), using a sampling pulse having a very short period, and then converted to a digital value at the analog-to-digital converter 34. On the other hand, a timer 36 starts time counting upon receipt of the send timing signal sent from the control unit 8 (CON), counts clock signals supplied from a clock generator 37 and outputs the counted value to a gate 39.

A received signal amplified by the amplifier 31 is also inputted to a reflected signal detector 38. The reflected signal detector 38, having a specified slice level, considers any received signal higher than the slice level to be a reflected signal and outputs a detection pulse. This detection pulse is supplied to the gate 39, causing the gate 39 to open. Therefore, reflected signal data from analog-to-digital converter 34 and time data from timer 36 are supplied to the memory unit 4 via the gate 39 at the timing when the detection pulse is output.

In Figure 4, the reflected signal data and time data corresponding to the beams of frequencies $f_1$ and $f_2$ stored in the memory unit 4 are read out to the operation unit 5. The operation unit 5 executes the operations of equations (9) and (11) using this data, then calculates and outputs an attenuation slope $\alpha$.

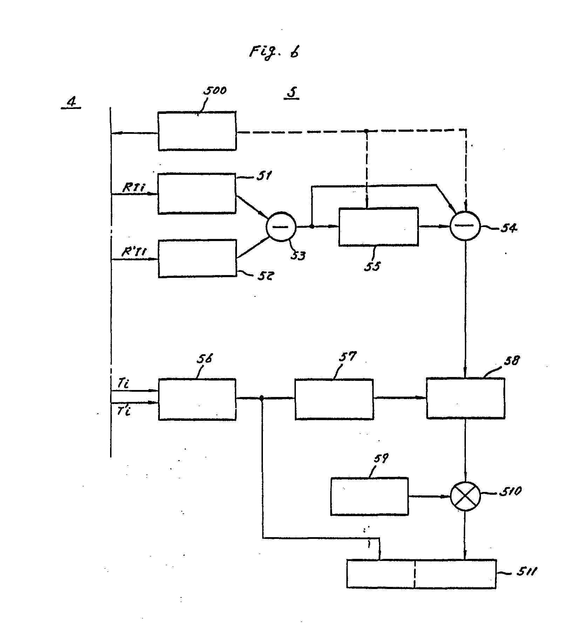

Figure 6 is a more detailed block diagram of an operation unit 5.

The operation unit 5 provides a generator 59 which generates a constant value "$4.343/V_0 \cdot (f_1-f_2)$", for evaluating the above equation (11), and is so configured that these constants are set for each frequency of the ultrasonic wave beams to be transmitted. A timing control circuit 500 extracts the reflected signal data, namely $Rli$, $R'li$ corresponding to respective beams, from memory unit 4, and the time data $Ti$. In other words, when analyzing the first reflected signal, $Rl_1$, $R'l_1$, $T_1$ are read out.

$Rl_1$, $R'l_1$, are input to natural logarithm circuits 51, 52 for logarithmic operations and then logarithmic values are subtracted by means of a subtraction circuit 53.

Thus, an output of the subtraction circuit 53 is the data indicating the values "$\ln (Rl_1/R'l_1)$". This data is supplied to a subtraction circuit 54.

Meanwhile, a register circuit 55 is initially reset by the timing control circuit 500 and outputs data indicating a value "0".

The subtraction circuit 54 subtracts the output data "0" of the register circuit 55 from the data indicating "$\ln (Rl_1/R'l_1)$" and therefore outputs data indicating the value "$\ln (Rl_1/R'l_1)$".

When the subtraction circuit 54 has completed subtraction, the output data of the subtraction circuit 53 is stored by the register circuit 55 for use in subsequent calculations.

8

Thus, in general the subtraction circuit 54 outputs $1_n (RI_i/R'I_i)-1_n(RI_{i-1}/R'I_{i-1})$. In the case of $1_n(RI_1/R'I_1)$, of course, the second term in this expression is zero, as indicated above.

On the other hand, time data Ti is read by a read-out circuit 56. A time-difference generation circuit 57 generates a value "$Ti-T_{i-1}$" corresponding to equation (11) and supplies the time $T_1$ (the time from send timing to the first reflected signal $RI_1$) to a divider circuit 58.

The divider circuit 58 divides the data indicating

$$"1n(RI_i/R'I_i)-1n(RI_{i-1}/R'I_{i-1})"$$

by the value "$T_i-T_{i-1}$".

The result of division is multiplied by the coefficient supplied from the abovementioned generator 59 by a multiplier circuit 510 and thereby the data is output as attenuation slope $\alpha$.

The attenuation slope $\alpha$ thus derived and the time data $T_i$, as data related to the period taken to travel from the sending location to the boundary between the domain OBi having the attenuation slope $\alpha$ and the next domain OBi+1, are set to the register 511 and then supplied to the display control unit 6 in Figure 6.

In Figure 4, the display control unit 6 identifies the tissue corresponding to the attenuation slope $\alpha$ on the basis of data transferred from the operation unit and carries out brightness modulation of a scan beam of an indicator or display screen 70.

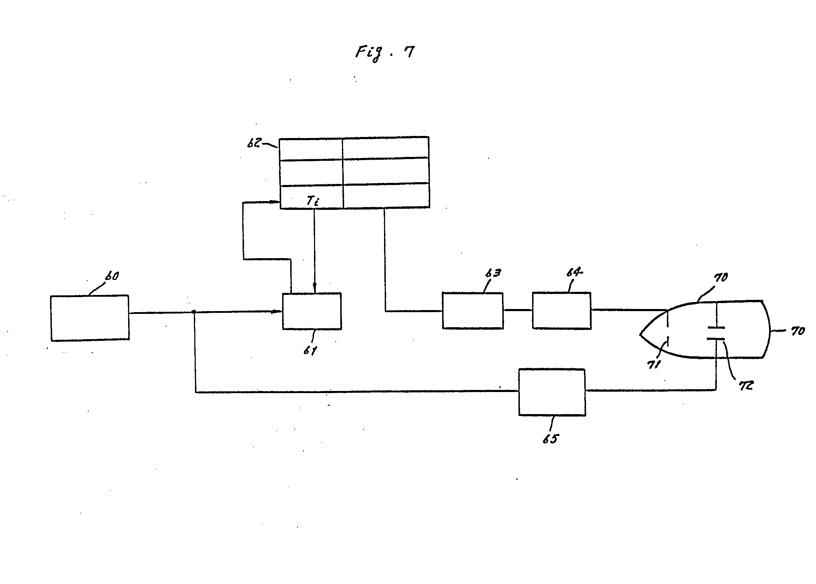

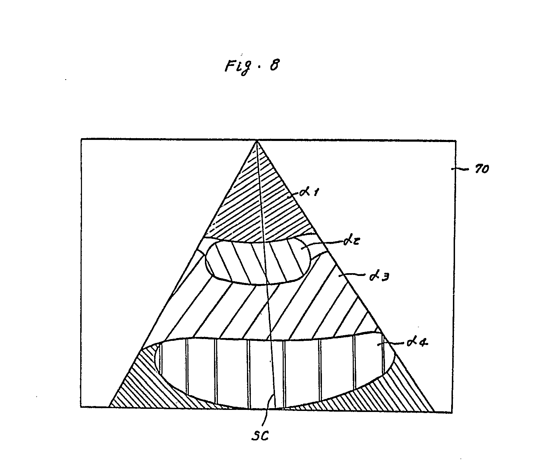

Figure 7 is a detailed block diagram of the display control unit 6 and Fig. 8 illustrates the display screen 70 of the display unit 7 showing a distribution diagram of attenuation.

The display control unit 6 includes a counter 60. When the counter 60 synchronizes the timer 36 of the receive unit 3 mentioned above, the scan period of one scanning line SC (see Figure 8) of the indicator 70 is in synchronization with the period over which reflected ultrasonic waves are received at the frequencies $f_1$ and $f_2$.

Namely, control is carried out in such a way that one scanning line SC is scanned during each send and receive period $T_s$ in the unit as indicated in Figure 3.

Operations of the unit 6 will be explained hereunder.

A count value is sequentially counted by the counter 60 and then supplied to a deflection control circuit 65. Thereby, the deflection control circuit 65 causes an electron beam of the display unit to scan by means of a beam deflection mechanism 72 of the display 7. The counted value of this counter 60 is then supplied to a comparator 61.

On the other hand, a buffer 62 which is composed of first-in/first-out registers accumulating the attenuation slope $\alpha$ and time data in the display control unit 6 in the sequence in which they are supplied is provided and the time data is supplied to the comparator 61.

In addition, the attenuation slope $\alpha$ is supplied to an attenuation constant table 63 and then converted to data for tissue identification. This converted data is then supplied to a digital-to-analog converter 64 and displayed on the display screen by means of a brightness controller 71. When the comparator 61 detects matching of count value and time data, it reads the next time data and attenuation constant pair stored in the buffer 62. This operation is repeated.

In this manner scanning is carried out on the display 7 in synchronization with the scanning of a specimen by the transducer 1.

Thereby, a tomographic image with domains $OB_1$, $OB_2$ ... shown in Figure 2 is obtained being discriminated by brightness or colour as indicated in Figure 8.

In the above embodiment, respective domains are discriminated by brightness or colour, but for example the biological tissue state identified may also be indicated or printed out. In this case, it is necessary to provide a memory corresponding to the displayed screen in the display control unit 6 and to add means for processing such as writing the identification data into the memory.

As explained previously, an embodiment of the present invention can provide for the accurate identification of the nature of respective domains in a specimen from reflected signals.

Thus, embodiments of the present invention provide a measurement method and system for measuring characteristics of attenuation of domains in an object by transmitting ultrasonic waves into the object and by receiving ultrasonic waves reflected from the object. The embodiments utilize signal intensities of reflected waves to measure attenuation characteristics. A plurality of ultrasonic waves of different frequencies are transmitted simultaneously or alternately to an object and the reflected waves are received from the object. The signal intensities corresponding to the transmission frequencies among the reflected waves are output and signal intensity ratios are also calculated. A signal intensity ratio indicates characteristics of attenuation, and from amongst the characteristics of attenuation an attenuation coefficient can be obtained using a time interval from a transmit time to a reflected wave receive time. An attenuation slope can be obtained from this attenuation coefficient and frequency difference between the transmitted ultrasonic waves.

Embodiments of this invention can be provided which are particularly suitable for investigation of the internal structure of the human body, but embodiments of this invention can also be employed for the investigation of other objects.

**0 041 403**

**Claims**

1. An ultrasonic wave method of measuring characteristics of attenuation of a domain or domains in an object, by transmitting an ultrasonic wave into the object and by receiving a reflected ultrasonic wave from the object, comprising:—

transmitting ultrasonic waves of a plurality of different frequencies into the object, and receiving ultrasonic waves reflected from the object, characterised by measuring characteristics of attenuation of the domain or domains from the ratio of ratios of intensities of received reflected ultrasonic waves corresponding to the respective different frequencies of the plurality.

2. A method as claimed in claim 1, wherein respective ultrasonic waves of respective different frequencies of the plurality are transmitted in a repeating succession.

3. A method as claimed in claim 1, wherein ultrasonic waves of the respective different frequencies of the plurality are transmitted simultaneously to the object, and wherein signals derived by transducer means from received reflected ultrasonic waves corresponding to the respective different frequencies of the plurality are separated for measuring the characteristics of attenuation.

4. A method as claimed in claim 1, 2 or 3, further comprising measuring a time interval or time intervals between ultrasonic wave transmission time or times and reflected wave reception time or times, and measuring the characteristics of attenuation from the measured time interval or time intervals and the intensity ratio or intensity ratios.

5. A method as claimed in any preceding claim, further comprising displaying images representing the measured characteristics of attenuation.

6. Ultrasonic wave apparatus for measuring characteristics of attenuation of a domain or domains in an object, by transmitting an ultrasonic wave into the object and by receiving a reflected ultrasonic wave from the object, comprising:—

ultrasonic wave transmit/receive means for transmitting ultrasonic waves of a plurality of different frequencies into the object and receiving ultrasonic waves reflected from the object, characterised by measurement means for measuring characteristics of attenuation of the domain or domains from the ratio or ratios of intensities of received reflected ultrasonic waves corresponding to the respective different frequencies of the plurality.

7. Apparatus as claimed in claim 6, wherein the ultrasonic wave transmit/receive means comprises an ultrasonic wave transducer means and a transmit circuit for causing the transducer means to transmit ultrasonic waves of the said plurality of different frequencies simultaneously.

8. Apparatus as claimed in claim 6, wherein the ultrasonic wave transmit/receive means comprises an ultrasonic wave transducer means and a transmit circuit for causing the transducer means to transmit respective ultrasonic waves of the respective different frequencies of the plurality in a repeating succession.

9. Apparatus as claimed in claim 7 or 8, wherein the ultrasonic wave transmit/receive means further comprises a receive circuit connected to the transducer means.

10. Apparatus as claimed in claim 9 when read as appended to claim 7, wherein the receive circuit is such that reflected ultrasonic waves corresponding to the respective different frequencies of the plurality can be separated upon receipt by the transducer means.

11. Apparatus as claimed in any one of claims 6 to 10, wherein the ultrasonic wave transmit/receive means comprises a transducer for use in common for ultrasonic wave transmission and reception.

12. Apparatus as claimed in any one of claims 6 to 10, wherein the ultrasonic wave transmit/receive means comprises a transmit-only transducer and a receive-only transducer.

13. Apparatus as claimed in any one of claims 6 to 12, wherein the ultrasonic wave transmit/receive means comprises a plurality of transducers corresponding to the respective different ultrasonic wave frequencies.

14. Apparatus as claimed in claim 12, wherein the receive-only transducer comprises an acousto-electric element which provides an output indicative of received wave intensity.

15. Apparatus as claimed in claim 9, wherein the receive circuit comprises a circuit which converts a reflected wave output from the transducer means into reflected wave intensity.

16. Apparatus as claimed in any one of claims 6 to 15, wherein the measurement means comprises a memory for storing data representative of reflected wave intensity and operation means connected to the memory for calculating sonic intensity ratio or ratios.

17. Apparatus as claimed in claim 9, wherein the receive circuit comprises a circuit which outputs data representative of a time interval from an ultrasonic wave transmission time to a reflected wave reception time.

10

18. Apparatus as claimed in claim 17, wherein the measurement means comprises a memory for storing data representative of reflected wave intensities and data representative of the time interval, and an operation circuit, which is connected to the memory, for calculating sonic intensity ratio or ratios and for calculating characteristics of attenuation utilising the time interval.

19. Apparatus as claimed in any one of claims 7 to 19, further comprising display means connected to the measurement means for displaying images representing measured characteristics of attenuation.

**Patentansprüche**

1. Verfahren zum Messen von Dämpfungseigenschaften einer Domäne oder von Domänen in einem Objekt mittels Ultraschallwellen, durch Senden einer Ultraschallwelle in das Objekt und durch Empfangen einer reflektierten Ultraschallwelle von dem Objekt, mit:

Senden einer Anzahl von Ultraschallwellen mit verschiedenen Frequenzen in das Objekt, und Empfangen von Ultraschallwellen, die von dem Objekt reflektiert worden sind, gekennzeichnet durch Messen von Dämpfungseigenschaften der Domäne oder der Domänen aus dem Verhältnis oder den Verhältnissen der Intensitäten der empfangenen reflektierten Ultraschallwellen, welche den jeweiligen verschiedenen Frequenzen entsprechen.

2. Verfahren nach Anspruch 1, bei welchem die jeweiligen Ultraschallwellen der Anzahl mit jeweils verschiedenen Frequenzen in wiederholender Reihenfolge gesendet werden.

3. Verfahren nach Anspruch 1, bei welchem die Anzahl von Ultraschallwellen mit jeweils verschiedenen Frequenzen gleichzeitig zu dem Objekt übertragen werden, und bei welchem Signale, die durch Übertrager von empfangenen reflektierten Ultraschallwellen abgeleitet werden, die der Anzahl von jeweils verschiedenen Frequenzen entsprechen, zur Messung der Dämpfungseigenschaften getrennt werden.

4. Verfahren nach Anspruch 1, 2 oder 3, dadurch gekennzeichnet, daß ein Zeitintervall oder Zeitintervalle zwischen der Übertragungszeit oder den Übertragungszeiten von Ultraschallwellen und der Empfangszeit oder den Empfangszeiten der reflektierten Wellen gemessen werden und die Dämpfungseigenschaften aus dem gemessenen Zeitintervall oder Zeitintervallen und dem Intensitätsverhältnis oder den Intensitätsverhältnissen gemessen werden.

5. Verfahren nach einem der vorhergehenden Ansprüche, ferner gekennzeichnet durch Displaybilder, welche die gemessenen Dämpfungseigenschaften darstellen.

6. Ultraschallwellenvorrichtung zum Messen der Dämpfungseigenschaft einer Domäne oder von Domänen in einem Objekt, durch Senden einer Ultraschallwelle in das Objekt und durch Empfangen einer von dem Objekt reflektierten Ultraschallwelle, mit:

einer Ultraschall-Sende/Empfangs-Einrichtung zum Senden von Ultraschallwellen mit einer Anzahl von verschiedenen Frequenzen in das Objekt und zum Empfangen von Ultraschallwellen, die von dem Objekt reflektiert worden sind, gekennzeichnet durch Meßeinrichtungen zum Messen der Dämpfungseigenschaft der Domäne oder der Domänen aus dem Verhältnis oder den Verhältnissen der Intensitäten der empfangenen reflektierten Ultraschallwellen, welche den jeweils verschiedenen Frequenzen entsprechen.

7. Vorrichtung nach Anspruch 6, bei welcher die Ultraschallwellen-Sende/Empfangs-Einrichtung eine Ultraschallwellen-Übertragungseinrichtung und eine Sendeschaltung umfaßt, um zu bewirken, daß die Übertragungseinrichtung Ultraschallwellen der genannten Anzahl von verschiedenen Frequenzen gleichzeitig sendet.

8. Vorrichtung nach Anspruch 6, bei welcher die Ultraschallwellen-Sende/Empfangs-Einrichtung eine Ultraschallwellen-Übertragungseinrichtung und eine Sendeschaltung umfaßt, um zu bewirken, daß die Übertragungseinrichtung jeweilige Ultraschallwellen der Anzahl von jeweils verschiedenen Frequenzen in wiederholender Reihenfolge sendet.

9. Vorrichtung nach Anspruch 7 oder 8, bei welcher die Ultraschallwellen-Sende/Empfangs-Einrichtung ferner eine mit der Übertragungseinrichtung verbundene Empfangsschaltung umfaßt.

10. Vorrichtung nach Anspruch 9 in Verbindung mit Anspruch 7, bei welcher die Empfangsschaltung so ist, daß reflektierte Ultraschallwellen, welche der Anzahl von jeweils verschiedenen Frequenzen entsprechen, bei Empfang durch die Übertragungseinrichtung getrennt werden können.

11. Vorrichtung nach einem der Ansprüche 6 bis 10, bei welcher die Ultraschallwellen-Sende/Empfangs-Einrichtung einen Übertrager umfaßt, der für die Aussendung und den Empfang von Ultraschallwellen gemeinsam benutzt wird.

12. Vorrichtung nach einem der Ansprüche 6 bis 10, bei welcher die Ultraschallwellen-Sende/Empfangs-Einrichtung einen nur sendenden Übertrager und einen nur empfangenen Übertrager umfaßt.

13. Vorrichtung nach einem der Ansprüche 6 bis 12 bei welcher die Ultraschallwellen-Sende/Empfangs-Einrichtung eine Vielzahl von Übertragern umfaßt, die den jeweils verschiedenen Frequenzen der Ultraschallwellen entsprechen.

14. Vorrichtung nach Anspruch 12, bei welcher der nur empfangende Übertrager ein akusto-elektrisches Element umfaßt, welches Ausgangzeichen liefert, die die empfangene Wellenintensität anzeigen.

· 15. Vorrichtung nach Anspruch 9, bei welcher die Empfangsschaltung eine Schaltung umfaßt, die einen reflektierten Wellenausgang von der Übertragungseinrichtung in Intensität der reflektierten Welle umwandelt.

16. Vorrichtung nach einem der Ansprüche 6 bis 15, bei welcher die Meßeinrichtung einen Speicher zur Speicherung der Daten umfaßt, die die Intensität der reflektierten Welle repräsentieren, und eine Betriebseinrichtung, die mit dem Speicher verbunden ist und zur Berechnung des Schallintensitätsverhältnisses oder der -verhältnisse dient.

17. Vorrichtung nach Anspruch 9, bei welcher die Empfangsschaltung eine Schaltung umfaßt, welche Daten ausgibt, die ein Zeitintervall von einer Ultraschallwellensendezeit zu einer Empfangszeit einer reflektierten Welle repräsentieren.

18. Vorrichtung nach Anspruch 17, bei welcher die Meßeinrichtung einen Speicher zur Speicherung von Daten umfaßt, die Intensitäten der reflektierten Wellen darstellen, und von Daten, welche das Zeitintervall darstellen, und ferner eine Betriebsschaltung, welche mit dem Speicher verbunden ist, um das Schallintensitätsverhältnis oder die -verhältnisse zu berechnen und um die Dämpfungseigenschaften unter Verwendung des Zeitintervalls zu berechnen.

19. Vorrichtung nach einem der Ansprüche 7 bis 18, ferner gekennzeichnet durch eine Anzeigereinrichtung, welche mit der Meßeinrichtung verbunden ist, um Bilder darzustellen, welche die gemessenen Dämpfungseigenschaften repräsentieren.

**Revendications**

1. Procédé de mesure par ondes ultrasonores des caractéristiques d'atténuation d'un ou plusieurs domaines dans un objet, en émettant une onde ultrasonore de l'objet et en recevant une onde ultrasonore réfléchie par l'objet, consistant à émettre des ondes ultrasonores de plusieurs fréquences différentes dans l'objet et à recevoir des ondes ultrasonores réfléchies par l'objet, caractérisé en ce qu'il consiste à mesurer des caractéristiques d'atténuation du domaine ou des domaines à partir du rapport ou des rapports des intensités des ondes ultrasonores réfléchies reçues correspondant aux différentes fréquences respectives.

2. Procédé selon la revendication 1, dans lequel des ondes ultrasonores respectives de différentes fréquences respectives sont émises dans une succession répétitive.

3. Procédé selon la revendication 1, dans lequel des ondes ultrasonores des différentes fréquences respectives sont émises simultanément vers l'objet et dans lequel des signaux délivrés par un dispositif transducteur à partir des ondes ultrasonores réfléchies reçues correspondant aux différentes fréquences respectives sont séparées pour mesurer les caractéristiques d'atténuation.

4. Procédé selon la revendication 1, 2 ou 3, consistant en outre à mesurer un intervalle de temps ou des intervalles de temps entre l'instant ou des instants d'émission d'ondes ultrasonores et l'instant ou les instants de réception des ondes réfléchies et à mesurer les caractéristiques d'atténuation à partir de l'intervalle de temps ou des intervalles de temps mesurés et le rapport ou les rapports d'intensités.

5. Procédé selon l'une quelconque des revendications précédentes, consistant en outre à visualiser des images représentant les caractéristiques d'atténuation mesurées.

6. Appareil de mesure par ondes ultrasonores des caractéristiques d'atténuation d'un ou plusieurs domaines dans un objet, en émettant une onde ultrasonore dans l'objet et en recevant une onde ultrasonore réfléchie par l'objet, comprenant un dispositif d'émission/réception d'ondes ultrasonores qui émet des ondes ultrasonores à plusieurs fréquences différentes dans l'objet et qui reçoit des ondes ultrasonores réfléchies par l'objet, caractérisé par un dispositif de mesure des caractéristiques d'atténuation du domaine ou des domaines à partir du rapport ou des rapports des intensités des ondes ultrasonores réfléchies reçues correspondant aux fréquences différentes respectives.

7. Appareil selon la revendication 6, dans lequel le dispositif d'émission/réception d'ondes ultrasonores consiste en un dispositif transducteur à ondes ultrasonores et en un circuit d'émission par lequel le dispositif transducteur émet simultanément lesdites plusieurs fréquences différentes.

8. Appareil selon la revendication 6, dans lequel le dispositif d'émission/réception d'ondes ultrasonores consiste en un dispositif transducteur à ondes ultrasonores et en un circuit d'émission par lequel le dispositif transducteur émet des ondes ultrasonores respectives des différentes fréquences respectives dans une succession répétitive.

9. Appareil selon la revendication 7 ou 8, dans lequel le dispositif d'émission/réception d'ondes ultrasonores comporte en outre un circuit de réception connecté au dispositif transducteur.

10. Appareil selon la revendication 9, considéré comme dépendant de la revendication 7, dans lequel le circuit de réception est tel que les ondes ultrasonores réfléchies correspondantes aux différentes fréquences respectives peuvent être séparées à la réception par le dispositif transducteur.

11. Appareil selon l'une quelconque des revendications 6 à 10, dans lequel le dispositif d'émission/réception d'ondes ultrasonores consiste en un transducteur utilisé en commun pour l'émission et la réception d'ondes ultrasonores.

12. Appareil selon l'une quelconque des revendications 6 à 10, dans lequel le dispositif d'émission/réception d'ondes ultrasonores consiste en un transducteur émetteur seulement et un transducteur récepteur seulement.

13. Appareil selon l'une quelconque des revendications 6 à 12, dans lequel le dispositif d'émission/réception d'ondes ultrasonores consiste en plusieurs transducteurs correspondant aux différentes fréquences respectives des ondes ultrasonores.

14. Appareil selon la revendication 12, dans lequel le transducteur de réception seulement consiste en un élément acoustique-électrique qui délivre une sortie indiquant l'intensité de l'onde reçue.

15. Appareil selon la revendication 9, dans lequel le circuit de réception consiste en un circuit qui convertit une sortie d'ondes réfléchies provenant du dispositif transducteur en une intensité d'ondes réfléchies.

16. Appareil selon l'une quelconque des revendications 6 à 15 dans lequel le dispositif de mesure consiste en une mémoire qui mémorise des données représentant une intensité d'ondes réfléchies et un dispositif d'opérations connectées à la mémoire pour calculer un rapport ou des rapports d'intensités acoustiques.

17. Appareil selon la revendication 9, dans lequel le circuit de réception consiste en un circuit qui émet des données représentant un intervalle de temps depuis un instant d'émission d'ondes ultrasonores jusqu'à un instant de réception d'ondes réfléchies.

18. Appareil selon la revendication 17, dans lequel le dispositif de mesure consiste en une mémoire qui mémorise des données représentant des intensités d'ondes réfléchies et des données représentant un intervalle de temps et un circuit d'opérations qui est connecté à la mémoire pour calculer un rapport ou des rapports d'intensité acoustique et pour calculer des caractéristiques d'atténuation en utilisant l'intervalle de temps.

19. Appareil selon l'une quelconque des revendications 7 à 19, comprenant en outre, un dispositif de visualisation connecté au dispositif de mesure pour visualiser des images représentant des carac-téristiques d'atténuation mesurées.

## Fig. 1

## Fig. 2

1

Fig. 3

Fig. 4

Fig. 5

0 041 403

Fig. 6

Fig. 7

$$Fig \cdot 8$$